# EUROPEAN PATENT SPECIFICATION

(11) **EP 3 784 659 B1**
(45) Date of publication and mention of the grant of the patent: **06.05.2026**
(21) Application number: 19720448.0
(22) Date of filing: 15.04.2019
(51) Int. Cl.: C07D 257/04

(54) **PROCESS FOR THE PREPARATION OF 1-(2-METHOXYMETHYL-3-METHYLPHENYL)4H-TETRAZOLIN-5-ONE**
VERFAHREN ZUR HERSTELLUNG VON 1-(2-METHOXYMETHYL-3-METHYLPHENYL)4H-TETRAZOLIN-5-ON
PROCÉDÉ DE PRODUCTION DE 1-(2-METHOXYMETHYL-3-METHYLPHENYL)4H-TÉTRAZOLIN-5-ONE

(30) Priority: 25.04.2018 EP 18169142
(43) Date of publication of application: 03.03.2021
(73) Proprietor: BASF SE, 67056 Ludwigshafen am Rhein (DE)
(72) Inventor: HALLER, Jan, 51379 Leverkusen (DE)
(74) Representative: BASF IP Association
(86) International application number: PCT/EP2019/059591
(87) International publication number: WO 2019/206695

(56) References cited:
- EP-A1- 0 711 761
- EP-A1- 2 990 404
- TAREK A. SALAMA ET AL: "Silicon-mediated Direct Conversion of Acyl Chlorides to Carbamoyl Azides or/and Tetrazolinones under Mild Conditions", CHEMISTRY LETTERS, vol. 40, no. 10, 5 October 2011 (2011-10-05), JAPAN, pages 1149 - 1151, XP055487745, ISSN: 0366-7022, DOI: 10.1246/cl.2011.1149

## Description

### TECHNICAL FIELD

The present invention relates to a process for the production of 1-(2-methoxymethyl-3-methylphenyl)4H-tetrazolin-5-one.

### TECHNICAL BACKGROUND

Agricultural crops, damaged or destroyed by pests, represent an important issue worldwide. In fact, the costs of such damage exceed billions annually due to decreased crop yields, reduced crop quality and increased harvesting costs. Therefore, a strong demand exists for the control of pests in agricultural crops by safe and effective means, which would have no harmful effects to mammals and other living organisms.

It has been disclosed in documents US 4956469, US 5003075 and US 5019152 that tetrazolinone derivatives exhibit pesticidal properties and can be useful as plant growth regulators.

Furthermore, it has been found that tetrazolinone derivatives exhibit interesting properties which can be useful in the fields of medicinal chemistry or electronics. For instance, document US 5705653 mentions that tetrazolinone derivatives can be used as β₃ agonists for the treatment of obesity and diabetes, while document US 2017/0200619 discloses the use of tetrazolinone derivatives for the removal of anti-reflective material and/or post-etch residue from micro electronic devices.

Documents EP2990404, US 4839349, US 5530135, WO 2005/112941, WO 2013/162072 and US 2014/364414 disclose the preparation of tetrazolinone derivatives by reacting an isocyanate compound with sodium azide in the presence of aluminum chloride in tetrahydrofuran (THF) or dimethylformamide (DMF). However, aluminum chloride is a very hygroscopic solid that reacts violently with water. Its addition to DMF, which is one of the preferred reaction solvents, provokes heat generation. The hydrochloric acid generated after the reaction of aluminum chloride with water can then react with sodium azide to generate the explosive hydrazoic acid. Furthermore, the aluminum salts resulting from the aqueous work-up tend to cause problems during filtration.

Documents US 5066667, US 5347010, WO 2004/80984, US 2011/130415, *"*Reactions of trimethylsilyl azide with heterocumulenes" (Journal of Organic Chemistry, 1980, 45, 5130), *"*Optically active antifungal azoles. VI. Synthesis and antifungal activity of N-[(1R,2R)-2-(2,4-Difluorophenyl)-2-hydroxy-1-methyl-3-(1H-1,2,4-triazol-1-yl)propyl]-N'-(4-substituted phenyl)-3(2H,4H)-1,2,4-triazolones and 5(1H,4H)-tetrazolones" (Chemical and Pharmaceutical Bulletin, 1996, 44, 314) and *"*Electronic properties of 1-methyl-4-phenyl-1H-tetrazole-5(4H)-thiones: An experimental and theoretical study" (Journal of Molecular Structure, 2009, 933, 38) disclose the preparation of tetrazolinone derivatives by reacting an isocyanate compound with a stoichiometric amount of trimethylsilyl azide either in benzene or without a solvent. Nevertheless, trimethylsilyl azide is a volatile compound, easily hydrolysable and highly toxic, which must be handled with great care in order to avoid inhalation and intoxication. In addition, trimethylsilyl azide is an expensive reagent and only 40% of its molar mass is actually found in the final product, the rest of the compound creating waste.

There is thus a need for novel methods for the production of 1-(2-methoxymethyl-3-methylphenyl)4H-tetrazolin-5-one, that overcome the abovementioned issues and offer a safe and efficient route to such compounds.

### SUMMARY OF THE INVENTION

It is a first object of the invention to provide a process for the preparation of 1-(2-methoxymethyl-3-methylphenyl)4H-tetrazolin-5-one (other name: 4-(2-methoxymethyl-3-methylphenyl)1H-tetrazolin-5-one with CAS number 1472649-96-9, herein also referred to as "the tetrazolinone"), comprising a step of reacting 2-methoxymethyl-3-methylphenylisocyanate (herein also referred as "the isocyanate compound") with an azide salt in a solvent and in the presence of a silicon derivative as a catalyst, wherein the silicon derivative is a trialkylsilyl azide, wherein the silicon derivative is prepared by reacting a trialkylsilyl halide compound with an azide salt, and wherein the silicon derivative is employed in a molar amount of less than 1.0 equivalent, relative to the isocyanate compound.

According to some embodiments, the azide salt is sodium azide.

According to some embodiments, the solvent is an amide, preferably selected from dimethylformamide, dimethylacetamide, N-methylpyrrolidone and combinations thereof.

The silicon derivative is a trialkylsilyl azide, preferably trimethylsilyl azide.

The silicon derivative is prepared by reacting a silyl halide compound with an azide salt.

According to some embodiments, the silyl halide compound is a trialkylsilyl halide.

According to some embodiments, the silyl halide compound is a silyl chloride compound.

According to some embodiments, the silyl halide compound is trimethylsilyl chloride.

The silicon derivative is employed in a molar amount of less than 1.0 molar equivalent, preferably less than 0.5 molar equivalent and more preferably less than 0.1 molar equivalent, relative to the isocyanate compound.

According to some embodiments, the step of reacting the isocyanate compound with the azide salt is performed at a temperature of at least 60°C, preferably of at least 80°C, and more preferably of at least 100°C.

According to some embodiments, a tetrazolinone salt is obtained after the step of reacting the isocyanate compound with the azide salt; and the process further comprises an additional step of treating the tetrazolinone salt with an acid to obtain the tetrazolinone compound.

According to some embodiments, the process comprises an intermediate purification step between the step of reacting the isocyanate compound with the azide salt and the additional step of treating the tetrazolinone salt with the acid.

According to some embodiments, the process further comprises a step of treating the tetrazolinone salt with sodium nitrite, before the additional step of treating the tetrazolinone salt with the acid, and preferably after the intermediate purification step.

The present invention makes it possible to address the need expressed above. In particular the invention provides a process for the production of tetrazolinones, that overcome the abovementioned issues and offer a safe and efficient route to such compounds.

This is achieved with the use of a silicon derivative as a catalyst, which is more easily handled and less hazardous than aluminum chloride. The invention also makes it possible to avoid using large quantities of expensive reagents, such as trimethylsilyl azide.

### DESCRIPTION OF EMBODIMENTS

The invention will now be described in more detail without limitation in the following description.

The present invention relates to a process for the preparation of 1-(2-methoxymethyl-3-methylphenyl)4H-tetrazolin-5-one (other name: 4-(2-methoxymethyl-3-methylphenyl)1H-tetrazolin-5-one with CAS number 1472649-96-9), comprising a step of reacting 2-methoxymethyl-3-methylphenylisocyanate ("the isocyanate compound") with an azide salt in a solvent and in the presence of a silicon derivative as a catalyst, wherein the silicon derivative is a trialkylsilyl azide, wherein the silicon derivative is prepared by reacting a trialkylsilyl halide compound with an azide salt, and wherein the silicon derivative is employed in a molar amount of less than 1.0 equivalent, relative to the isocyanate compound.

The azide salt can be notably chosen from sodium azide, potassium azide, lithium azide, or ammonium azide. The ammonium azide can notably be a quaternary ammonium azide salt, such as a tetraalkylammonium azide.

Examples of tetraalkylammonium azides are tetramethylammonium azide, tetraethylammonium azide, and tetrabutylammonium azide.

The azide salt is preferably sodium azide.

The solvent is preferably a polar solvent. Preferably the solvent comprises an amide such as dimethylformamide, dimethylacetamide or N-methylpyrrolidone.

According to some (preferred) embodiments, the solvent used in the process consists of a single compound chosen from the abovementioned compounds.

According to the present invention, the silicon derivative is used as a catalyst, which means that the silicon derivative is substantially not consumed during the step of reacting the isocyanate compound with the azide salt.

According to some embodiments, the silicon derivative that is used as a catalyst can have the general formula (II):

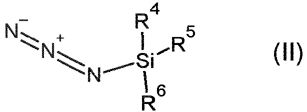

In formula (II), each of R⁴, R⁵ and R⁶ can independently be selected from alkyl groups.

Each of R⁴, R⁵ and R⁶ can notably be chosen from a methyl group, an ethyl group, an isopropyl group or a tert-butyl group .

R⁴, R⁵ and R⁶ can be the same or different.

If the substituents R⁴, R⁵ and R⁶ are the same, they can be for example methyl groups (trimethylsilyl azide), ethyl groups (triethylsilyl azide) or isopropyl groups (triisopropylsilyl azide).

If R⁴, R⁵ and R⁶ are different, R⁴ and R⁵ can be for example a methyl group and R⁶ can be an isopropyl group (isopropyldimethylsilyl azide), or R⁴ and R⁵ can be an ethyl group and R⁶ can be an isopropyl group (diethyliisopropylsilyl azide), or R⁴ and R⁵ can be a methyl group and R⁶ can be a tert-butyl group (tert-butyldimethylsilyl azide).

Preferably, the silicon derivative is trimethylsilyl azide.

In some embodiments, the silicon derivative is prepared prior to or during the step of reacting the isocyanate compound with the azide salt. The preparation may be performed in the same reaction vessel used for reacting the isocyanate compound with the azide salt.

The preparation of the silicon derivative is made by reacting a silyl halide compound with an azide salt. The azide salt used in this preparation step can notably be as defined above. It can be the same azide salt also used for the reaction with the isocyanate compound or a different one. Preferably it is the same. Sodium azide is a preferred salt for preparing the silicon derivative.

According to some embodiments, the silyl halide compound can have the general formula (III):

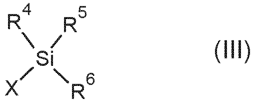

R⁴, R⁵ and R⁶ are as defined above.

X is a halogen, preferably selected from chlorine, bromine and iodine. The silyl halide compound can be chosen from a silyl chloride compound, or a silyl bromide compound, or a silyl iodide compound. Preferably, the silyl halide compound is a silyl chloride compound, and more preferably, the silyl halide compound is a trialkylsilyl chloride, most preferably trimethylsilyl chloride.

According to some embodiments, the silicon derivative can be prepared by the addition of the azide salt to a mixture containing the silyl halide and the solvent.

Alternatively and preferably, the silicon derivative can be prepared by the addition of the silyl halide to a mixture containing the azide salt and the solvent.

The isocyanate compound may be added subsequently or may be present in the above mixture containing the silyl halide and solvent or containing the azide salt and the solvent.

The silicon derivative is present in a molar amount of less than 1.0 equivalent, preferably less than 0.5 equivalent and more preferably less than 0.1 equivalent, relative to the isocyanate compound.

The silicon derivative can for example be employed in a molar amount from 0.9 to 0.99 equivalent, or from 0.8 to 0.9 equivalent, or from 0.7 to 0.8 equivalent, or from 0.6 to 0.7 equivalent, or from 0.5 to 0.6 equivalent, or from 0.4 to 0.5 equivalent, or from 0.3 to 0.4 equivalent, or from 0.2 to 0.3 equivalent, or from 0.1 to 0.2 equivalent, or from 0.075 to 0.1 equivalent, or from 0.05 to 0.075 equivalent, or from 0.025 to 0.05 equivalent, or from 0.01 to 0.025 equivalent, relative to the isocyanate compound.

According to some embodiments, the azide salt can be present in a molar amount of from 0.5 to 5.0 equivalent, relative to the isocyanate compound. Preferably, the azide salt is present in a molar amount of at least 1.0 equivalent, or at least 1.01 equivalent, or at least 1.02 equivalent, or at least 1.03 equivalent, or at least 1.04 equivalent, or at least 1.05 equivalent, relative to the isocyanate compound.

The azide salt can for example be employed in a molar amount from 0.5 to 0.9 equivalent, or from 0.9 to 1.0 equivalent, or from 1.0 to 1.1 equivalent, or from 1.1 to 1.2 equivalent, or from 1.2 to 1.5 equivalent, or from 1.5 to 2.0 equivalent, or from 2.0 to 5.0 equivalent, relative to the isocyanate compound.

The above amounts correspond to the reactants initially put into contact, after the optional step of preparing the silicon derivative.

According to some embodiments, the step of reacting the isocyanate compound with the azide salt can be performed at a temperature of at least 60°C, preferably of at least 80°C, and more preferably of at least 100°C.

The step of reacting the isocyanate compound with the azide salt can notably be performed at a temperature from 60 to 70°C, or from 70 to 80°C, or from 80 to 90°C, or from 90 to 100°C, or from 100 to 110°C, or from 110 to 120°C, or from 120 to 130°C, or from 130 to 140°C, or from 140 to 150°C.

According to some embodiments, the isocyanate compound is added to a reaction mixture comprising the azide salt and/or the silicon derivative. Said addition can in particular be carried out in less than 2 hours, more preferably in less than 1.5 hours, more preferably in less than 1 hour, more preferably in less than 50 minutes, and more preferably in less than 45 minutes.

According to some embodiments, the isocyanate compound may be diluted with an inert solvent such as toluene for example, and this mixture may be then added to the reaction mixture.

According to some embodiments, after the step of reacting the isocyanate compound with the azide salt, a tetrazolinone salt is obtained.

In particular, the tetrazolinone salt may have the general formula (IV):

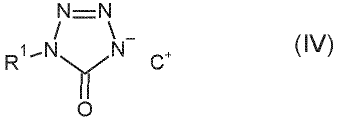

R¹ is 2-methoxymethyl-3-methylphenyl .

C⁺ is a cation coming from the cation present in the azide salt, and it can be notably chosen from sodium, potassium, lithium and ammonium ions. The ammonium ions can notably be quaternary ammonium ions, such as tetraalkylammonium ions.

Examples of tetraalkylammonium ions are tetramethylammonium ions, tetraethylammonium ions, and tetrabutylammonium ions.

Preferably, C⁺ is a sodium ion.

The desired tetrazolinone compound can be the above tetrazolinone salt. In this case, the tetrazolinone salt, which is soluble in the solvent in this step, can be recovered, and optionally washed and/or purified, e.g. by filtration. The aim of the filtration is to remove solid by-products.

Alternatively, the above tetrazolinone salt may be further converted to the desired tetrazolinone compound. Accordingly, the process may further comprise an additional step of treating the tetrazolinone salt with an acid. The acid may for instance directly be added to the reaction mixture obtained from the step of reacting the isocyanate compound with the azide salt. Alternatively, the tetrazolinone salt, which is soluble in the solvent in this step, may be filtered to remove solid by-products, prior to being treated with the acid.

The acid used to convert the tetrazolinone salt to the tetrazolinone compound can notably be used in an amount such that the pH of the medium containing the tetrazolinone salt is reduced to less than 3, or less than 2, or less than 1.5, or more preferably less than 1. Such acid can be either an inorganic or an organic acid. Examples of inorganic acids are: hydrochloric acid, hydrobromic acid, hydroiodic acid, sulfuric acid, phosphoric acid, carbonic acid, and nitric acid. Examples of organic acids are: trifluoroacetic acid, trichloroacetic acid, methanesulfonic acid, ethane sulfonic acid, benzenesulfonic acid, p-toluenesulfonic acid, trifluoromethanesulfonic acid, and formic acid. Combinations of the above acids can also be used. Preferably, the acid used to transform the tetrazolinone salt to the tetrazolinone compound is hydrochloric acid or sulfuric acid.

According to some embodiments, after filtration of the solid by-products and prior to being treated with the acid, the tetrazolinone salt can be treated with a substance such as e.g. sodium nitrite in order to provoke the decomposition of residual azide. The amount of sodium nitrite may be adjusted so as to fully decompose the residual azide.

Sodium nitrite can preferably be used in a molar amount of from 0.01 to 1.0 equivalent, relative to the isocyanate compound. In some embodiments, sodium nitrite is used in a molar amount of less than 1.0 equivalent, or less than 0.9 equivalent, or less than 0.8 equivalent, or less than 0.7 equivalent, or less than 0.6 equivalent, or less than 0.5 equivalent, or less than 0.4 equivalent, or less than 0.3 equivalent, or less than 0.2 equivalent, or less than 0.1 equivalent, or less than 0.05, or less than 0.02 equivalent, or equal to 0.01 equivalent, relative to the isocyanate compound.

The desired tetrazolinone compound can then be recovered, and optionally washed and/or purified, e.g. by filtration.

The desired tetrazolinone can also be used in another reaction for the synthesis of tetrazolinone derivatives, as for example described in US 4839349.

The tetrazolinone compound thus obtained has the general formula (V):

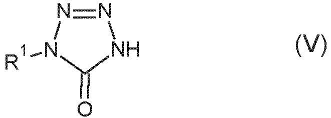

R¹ is 2-methoxymethyl-3-methylphenyl .

By way of illustration, sodium azide can be used as the azide salt and trimethylsilyl chloride can be used as the silyl halide derivative to form the silicon derivative, which in this case is trimethylsilyl azide. The corresponding reaction scheme is shown below.

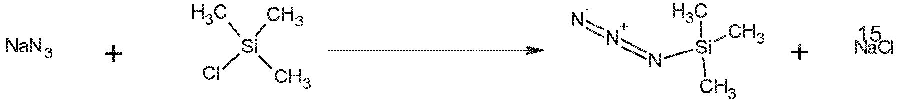

Trimethylsilyl azide can then react with the isocyanate compound, to give an intermediate trimethylsilyl tetrazolinone which after reaction with sodium azide can lead to the formation of the tetrazolinone sodium salt and trimethylsilyl azide which is thus regenerated, as shown below.

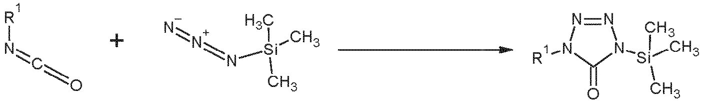

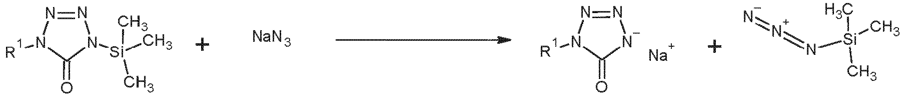

The tetrazolinone salt can either be isolated as is or, alternatively and preferably, the tetrazolinone salt can be treated with an acid (for example hydrochloric acid) to give the tetrazolinone compound having the structure (V), as shown below.

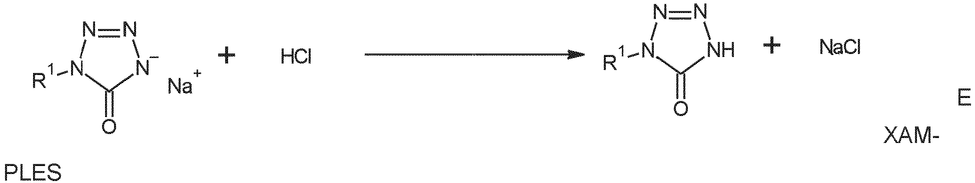

The following examples illustrate the invention without limiting it.

### Example 1:

To 10.6 mL of dimethylformamide were added 0.20 g (2.5 mol%) of trimethylsilyl chloride and 4.82 g (1.03 equiv) of sodium azide. The mixture was stirred at 80°C and 13.2 g (1.0 equiv) of 2-methoxymethyl-3-methylphenylisocyanate were added over 45 min. The suspension was stirred for 1.3 h further at 80°C, cooled at 30°C, and to the mixture were slowly added (30 min) 35 mL water and 6.5 g of a sodium hydroxide solution, 25%. The solid by-products were removed by filtration and washed with water (7 mL). 1 g of an aqueous solution of sodium nitrite, 40%, was added to the filtrate, followed by the addition of 13.1 g of concentrated hydrochloric acid (36-37%). The suspension was then filtered and washed with water until the pH of the filtrate was adjusted to 3. After drying, 1-(2-methoxymethyl-3-methylphenyl)4H-tetrazolin-5-one was obtained (14.4 g) in 86% yield, with a purity of 98.3 area% (HPLC) and with an assay of 98.0% w/w.

### Example 2:

To 154 g of dimethylformamide were added 3 g (2.8 mol%) of trimethylsilyl chloride and 72 g (0.98 equiv) of sodium azide. The mixture was stirred at 100°C and a mixture of 200 g (1.0 equiv) of 2-methoxymethyl-3-methylphenylisocyanate and 22 g of toluene were added over 1.3 h. The suspension was stirred for 2.5 h further at 100°C, cooled at 20°C, and to the mixture were slowly added (30 min) 619 g of sodium hydroxide 3.9% in water. The solid by-products were removed by filtration and 15 g of an aqueous solution of sodium nitrite, 40% were added to the filtrate. To the filtrate were then added at 20 to 50°C a mixture of 196 g of concentrated hydrochloric acid (36-37%) and of 520 mL of water. This was followed by the further addition of 32 g of concentrated hydrochloric acid (36-37%). The suspension was then filtered (at 20°C) and washed with 810 mL of water to yield 735 g water wet 1-(2-methoxymethyl-3-methylphenyl)4H-tetrazolin-5-one with an assay of 28.7% w/w (87% yield).

## Claims

1. A process for the preparation of 1-(2-methoxymethyl-3-methylphenyl)4H-tetrazolin-5-one, comprising a step of reacting 2-methoxymethyl-3-methyl phenyl isocyanate with an azide salt in a solvent and in the presence of a silicon derivative as a catalyst, wherein the silicon derivative is a trialkylsilyl azide, wherein the silicon derivative is prepared by reacting a trialkylsilyl halide compound with an azide salt, and wherein the silicon derivative is employed in a molar amount of less than 1.0 equivalent, relative to the isocyanate compound.

2. The process according to claim 1, wherein the azide salt is sodium azide.

3. The process according to claim 1 or 2, wherein the solvent is an amide, preferably selected from dimethylformamide, dimethylacetamide, N-methylpyrrolidone and combinations thereof.

4. The process according to any one of claims 1 to 3, wherein the silicon derivative is trimethylsilyl azide.

5. The process according to any one of claims 1 to 4, wherein the silyl halide compound is a silyl chloride compound.

6. The process according to claim 5, wherein the silyl halide compound is trimethylsilyl chloride.

7. The process according to any one of claims 1-6, wherein the silicon derivative is employed in a molar amount of less than 0.5 equivalent and more preferably less than 0.1 equivalent, relative to the isocyanate compound.

8. The process according to any one of claims 1-7, wherein the step of reacting the isocyanate compound with the azide salt is performed at a temperature of at least 60°C, preferably of at least 80°C, and more preferably of at least 100°C.

9. The process according to any one of claims 1-8, wherein a tetrazolinone salt is obtained after the step of reacting the isocyanate compound with the azide salt; and the process further comprises an additional step of treating the tetrazolinone salt with an acid to obtain the tetrazolinone compound.

10. The process according to claim 9, comprising an intermediate purification step between the step of reacting the isocyanate compound with the azide salt and the additional step of treating the tetrazolinone salt with the acid.

11. The process according to claim 9 or 10 further comprising a step of treating the tetrazolinone salt with sodium nitrite, before the additional step of treating the tetrazolinone salt with the acid, and preferably after the intermediate purification step.

## Patentansprüche

1. Verfahren zur Herstellung von 1-(2-Methoxymethyl-3-methylphenyl)-4H-tetrazolin-5-on, umfassend einen Schritt des Umsetzens von 2-Methoxymethyl-3-methylphenylisocyanat mit einem Azidsalz in einem Lösungsmittel und in Gegenwart eines Siliciumderivats als Katalysator, wobei es sich bei dem Siliciumderivat um ein Trialkylsilylazid handelt, wobei das Siliciumderivat durch Umsetzen einer Trialkylsilylhalogenidverbindung mit einem Azidsalz hergestellt wird und wobei das Siliciumderivat in einer molaren Menge von weniger als 1,0 Äquivalent, bezogen auf die Isocyanatverbindung, eingesetzt wird.

2. Verfahren nach Anspruch **1,** wobei es sich bei dem Azidsalz um Natriumazid handelt.

3. Verfahren nach Anspruch 1 oder 2, wobei es sich bei dem Lösungsmittel um ein Amid handelt, das vorzugsweise aus Dimethylformamid, Dimethylacetamid, **N-**Methylpyrrolidon und Kombinationen davon ausgewählt wird.

4. Verfahren nach einem der Ansprüche 1 bis 3, wobei es sich bei dem Siliciumderivat um Trimethylsilylazid handelt.

5. Verfahren nach einem der Ansprüche 1 bis 4, wobei es sich bei der Silylhalogenidverbindung um eine Silylchloridverbindung handelt.

6. Verfahren nach Anspruch 5, wobei es sich bei der Silylhalogenidverbindung um Trimethylsilylchlorid handelt.

7. Verfahren nach einem der Ansprüche 1-6, wobei das Siliciumderivat in einer molaren Menge von weniger als 0,5 Äquivalent und weiter bevorzugt weniger als 0,1 Äquivalent, bezogen auf die Isocyanatverbindung, eingesetzt wird.

8. Verfahren nach einem der Ansprüche 1-7, wobei der Schritt des Umsetzens der Isocyanatverbindung mit dem Azidsalz bei einer Temperatur von mindestens 60 °C, vorzugsweise von mindestens 80 °C und weiter bevorzugt von mindestens 100 °C durchgeführt wird.

9. Verfahren nach einem der Ansprüche 1-8, wobei nach dem Schritt des Umsetzens der Isocyanatverbindung mit dem Azidsalz ein Tetrazolinonsalz erhalten wird und das Verfahren ferner einen zusätzlichen Schritt des Behandelns des Tetrazolinonsalzes mit einer Säure zum Erhalt der Tetrazolinonverbindung umfasst.

10. Verfahren nach Anspruch 9, umfassend einen Zwischenreinigungsschritt zwischen dem Schritt des Umsetzens der Isocyanatverbindung mit dem Azidsalz und dem zusätzlichen Schritt des Behandelns des Tetrazolinonsalzes mit der Säure.

11. Verfahren nach Anspruch 9 oder 10, ferner umfassend einen Schritt des Behandelns des Tetrazolinonsalzes mit Natriumnitrit vor dem zusätzlichen Schritt des Behandelns des Tetrazolinonsalzes mit der Säure und vorzugsweise nach dem Zwischenreinigungsschritt.

## Revendications

1. Procédé de préparation de 1-(2-méthoxyméthyl-3-méthylphényl)4H-tétrazolin-5-one, comprenant une étape de mise en réaction de 2-méthoxyméthyl-3-méthylphénylisocyanate avec un sel d'azoture dans un solvant et en présence d'un dérivé de silicium comme catalyseur, dans lequel le dérivé de silicium est un azoture de trialkylsilyle, dans lequel le dérivé de silicium est préparé par mise en réaction d'un composé halogénure de trialkylsilyle avec un sel d'azoture, et dans lequel le dérivé de silicium est utilisé en une quantité molaire inférieure à 1,0 équivalent par rapport au composé isocyanate.

2. Procédé selon la revendication 1, dans lequel le sel azoture est l'azoture de sodium.

3. Procédé selon la revendication 1 ou 2, dans lequel le solvant est un amide, de préférence choisi parmi le diméthylformamide, le diméthylacétamide, la N-méthylpyrrolidone et leurs combinaisons.

4. Procédé selon l'une quelconque des revendications 1 à 3, dans lequel le dérivé de silicium est l'azoture de triméthylsilyle.

5. Procédé selon l'une quelconque des revendications 1 à 4, dans lequel le composé halogénure de silyle est un composé chlorure de silyle.

6. Procédé selon la revendication 5, dans lequel le composé halogénure de silyle est le chlorure de triméthylsilyle.

7. Procédé selon l'une quelconque des revendications 1 à 6, dans lequel le dérivé de silicium est utilisé en une quantité molaire de moins de 0,5 équivalent et plus préférablement de moins de 0,1 équivalent, par rapport au composé isocyanate.

8. Procédé selon l'une quelconque des revendications 1 à 7, dans lequel l'étape de mise en réaction du composé isocyanate avec le sel d'azoture est effectuée à une température d'au moins 60 °C, de préférence d'au moins 80 °C, et plus préférentiellement d'au moins 100 °C.

9. Procédé selon l'une quelconque des revendications 1 à 8, dans lequel un sel de tétrazolinone est obtenu après l'étape de mise en réaction du composé isocyanate avec le sel d'azoture ; et le procédé comprend en outre une étape supplémentaire de traitement du sel de tétrazolinone par un acide pour obtenir le composé de tétrazolinone.

10. Procédé selon la revendication 9, comprenant une étape de purification intermédiaire entre l'étape de mise en réaction du composé isocyanate avec le sel d'azoture et l'étape supplémentaire de traitement du sel de tétrazolinone par l'acide.

11. Procédé selon la revendication 9 ou 10, comprenant en outre une étape de traitement du sel de tétrazolinone avec du nitrite de sodium, avant l'étape supplémentaire de traitement du sel de tétrazolinone par l'acide, et de préférence après l'étape de purification intermédiaire.
